# EUROPÄISCHE PATENTSCHRIFT

(11) **EP 1 887 899 B1**
(45) Veröffentlichungstag und Bekanntmachung des Hinweises auf die Patenterteilung: **16.09.2015**
(21) Anmeldenummer: 06742582.7
(22) Anmeldetag: 13.04.2006
(51) Int. Cl.: A43B 7/14, A43B 7/22, A43B 17/02, A61F 5/14, A43B 1/00

(54) **ORTHOPÄDISCH ZUGERICHTETER SCHUH**
ORTHOPEDICALLY FINISHED FOOTWEAR
CHAUSSURE A ADAPTATION ORTHOPEDIQUE

(30) Priorität: 13.04.2005 DE 102005017100
(43) Veröffentlichungstag der Anmeldung: 20.02.2008
(73) Patentinhaber: Hallufix AG, 81925 München (DE)
(72) Erfinder: Krauss, Axel, 81541 München
(74) Vertreter: Thoma, Michael
(86) Internationale Anmeldenummer: PCT/EP2006/003425
(87) Internationale Veröffentlichungsnummer: WO 2006/108660

(56) Entgegenhaltungen:
- EP-A- 0 549 539
- WO-A-00/70984
- US-A- 4 316 333
- US-A- 4 841 648
- US-B1- 6 227 458

## Beschreibung

Die Erfindung betrifft eine Vorrichtung und ein Verfahren zum orthopädischen Zurichten von Schuhwerk, insbesondere zur Verwendung in einer orthopädiemechanischen Werkstatt.

Unter orthopädischem Zurichten von Schuhwerk im Sinne der Erfindung ist zu verstehen, Konfektionsschuhe durch Abändern der Innenschuhsohlenkontur individuell auf einen Patientenfuß anzupassen und das Schuhwerk somit zu individualisieren. Derzeit ist es üblich, Konfektionsschuhe patientenindividuell durch handwerkliches Umarbeiten der Schuhinnensohle zuzurichten. Hierbei werden Korrekturelemente wie z. B. Pelotten, retrokapitale Abstützungen, Zehenstrecker, Längs- und/oder Quergewölbestützen, Supinationsleisten oder Pronationsleisten patientenindividuell hergestellt oder aus einem vorhandenen Sortiment passend ausgewählt. Um die Korrekturelemente patientenindividuell einarbeiten zu können, müssen erst die Füße der Patienten abgemessen werden. Hierfür werden üblicherweise Gipsabdrücke, Trittschaumabdrücke oder Blaupausenabdrücke von den Patientenfüßen gemacht. Die Fußabdrücke helfen den Orthopädie-Technikern bzw. Orthopädie - Schuhtechnikern die Korrekturelemente an die richtige Stelle zu positionieren. Damit die konfektionierten Schuhe patientenindividuell umgearbeitet werden können, müssen die Schuhe in der orthopädischen Werkstatt zurückgelassen werden. Der Techniker arbeitet dann nach den vorher genommenen Abdrücken, die Schuhe um. Hierfür wird, im Rahmen einer aufwändigen handwerklichen Arbeitsprozedur, die Innensohle der konfektionierten Schuhe herausgetrennt und alle Klebstoffreste entfernt und Unebenheiten geglättet. Dann werden die für den Patienten benötigten Korrekturelemente eingearbeitet. Sitzen die Pelotten an der richtigen Stelle, wird noch eine neue Lederdecke als Decksohle in die Schuhe eingeklebt. Nach diesen Arbeitsschritten ist der patientengerechte Umbau des konfektionierten Schuhes beendet und der Patient erhält seine Schuhe zurück. Bei dieser Vorgehensweise ist von Nachteil, dass die gesamte Prozedur wiederholt werden muss, sofern eines der Korrekturelemente auch nur geringfügig falsch positioniert ist und beim Patienten Unwohlsein, z. B. Druckstellen oder dergleichen hervorruft. Außerdem ist diese handwerkliche Zurichtung von Konfektionsschuhwerk aufwändig und teuer. Dieser Aufwand (circa € 100,00 pro Schuhpaar) wird in absehbarer Zeit von den gesetzlichen Krankenkassen nicht mehr übernommen werden.

Außerdem ist es bekannt, Korrekturelemente mittels einer Klebschicht direkt auf die Innensohle des zuzurichtenden Schuhwerks aufzukleben. Dieses Vorgehen hat den Nachteil, dass die Klebeverbindung zwischen den Korrekturelementen und der Schuhinnensohle oftmals von unbefriedigender Festigkeit ist und insbesondere unter Einwirkung der Fußwärme und/oder -feuchtigkeit der Klebstoff erweicht und ein Verrutschen des oder der Korrekturelemente zu beobachten ist. Weiterhin ist von Nachteil, dass ein einmal eingeklebtes Korrekturelement meist nicht ohne Zerstörung der Klebverbindung in seiner Position korrigiert werden kann. Dies macht es erforderlich, die Korrekturelemente bereits beim ersten Versuch korrekt zu positionieren. Erfolgt dies nicht, wird derzeit das falsch eingeklebte Korrekturelement entfernt und weggeworfen und ein neues Korrekturelement mit einer neuen Klebschicht an eine geänderte Position geklebt. Das abgetrennte oder herausgetrennte Korrekturelement ist Abfall, was zu erheblichen Kosten führt. Weiterhin ist bei einer direkten Klebverbindung zwischen der Korrekturvorrichtung und der Innensohle von Nachteil, dass der Klebstoff auch unter Wärme- und Feuchtigkeitseinwirkung eine hohe Festigkeit zwischen dem Material der Innensohle (Leder, Textilien, Kunststoffe oder dergleichen) und dem Material der Korrekturelemente, die aus Moosgummi, Polyethylen- und Polyurethanschaumstoffen hergestellt sind, erbringen muss. In der Praxis hat sich jedoch gezeigt, dass dies mit üblichen Klebstoffen nicht immer erreichbar ist, so dass sich die Korrekturelemente mit der Zeit unter Wärme- und/oder Feuchtigkeitseinwirkung im Schuh verschieben und somit an eine ungeeignete Position gelangen.

Weiterhin ist es aus der DE 203 14 286 U1 eine mehrteilige Fußeinlage bekannt, die ein Mittelfußteil aufweist, an welches unterschiedliche Vorderfuß und Fersenteile anfügbar sind. Somit kann eine patientenindividuell abgestimmte Fußeinlage hergestellt werden. Fußeinlagen sind jedoch zum orthopädischen Zurichten von Schuhwerk nicht geeignet, da diese einen hohen Platzbedarf aufweisen und insbesondere in engem modischem Schuhwerk vom Patienten als nicht tragbar empfunden werden. Außerdem ist das Herstellen und Anpassen einer solchen mehrteiligen Fußeinlage aufwändig und teuer. Patientenindividuell angepasste Fußeinlagen können somit das Zurichten von insbesondere modischem Schuhwerk nicht ersetzen. Weiterhin beeinflussen solche Fußeinlagen durch ein formstabiles, d. h. starres Mittelteil das Abrollverhalten des Schuhs negativ. So wird mit einer Fußeinlage gem. der DE 203 14 286 41 der Schuh in seiner Längsrichtung versteift. Dies kann bei einem Schuh, der nicht besonders fest am Fuß sitzt, z. B. einem Slipper, dazu führen, dass der Schuh beim Laufen von der Ferse abrutscht (sog. "Schlappen" des Schuhs). Des weiteren ist bei solchen Fußeinlagen zur Anpassung nahezu immer eine Nacharbeit durch einen Orthopädietechniker notwendig.

Für die Verwendung in Schuhen mit hohen Absätzen, die also eine hohe Sprengung aufweisen, ist eine solche Fußeinlage gänzlich ungeeignet, da durch das starre Mittelteil der Fuß des Patienten/der Patientin ausgehebelt wird, wenn diese an der steilen Schräge eines hohen Absatzschuhs anliegt.

Aus dem deutschen Gebrauchsmuster DE 298 10 518 U1 ist ein Baukastensystem für eine Fußeinlage bekannt, bei dem eine Vielzahl von Einlagengrundkörpern verwendet werden, an die verschiedene Ergänzungsteile geheftet werden. Auch ein solches Baukastensystem für eine Schuheinlage ist für das orthopädische Zurichten von Konfektionsschuhwerk nicht geeignet, da ein erheblicher höhenmäßiger Platzbedarf vorhanden ist und zudem ungewollte Stoßkanten zwischen dem Einlagengrundkörper und angehefteten Einlagenerweiterungen vorhanden sind. Diese werden vom Patienten als unkomfortabel empfunden. Ein solches Baukastensystem für Fußeinlagen kann somit ebenfalls das Zurichten von Schuhwerk, insbesondere von modischem Schuhwerk, z. B. mit hohen Absätzen, nicht ersetzen.

Ein orthopädisch zugerichteter Schuh gemäss dem Oberbegriff des Anspruchs 1 ist aus der Druckschrift WO 00/70984 bekannt.

Ausgehend hiervon besteht das Bedürfnis, eine Vorrichtung zum orthopädischen Zurichten von Schuhwerk anzugeben, welche nur wenige Teile umfasst, sehr kostengünstig in der Herstellung ist und zudem an individuell anatomische Gegebenheiten eines Patientenfußes anpassbar, insbesondere korrigierbar ist. Insbesondere soll die Vorrichtung zum orthopädischen Zurichten von konfektionierten Schuhwerk eine aufwändige handwerkliche Bearbeitung des Schuhs zum Zwecke der individuellen Anpassung an den Patientenfuß vermeiden und für eine Vielzahl verschiedener Arten von Konfektionsschuhen, z. B. auch modisches Damenschuhwerk, wie Pumps mit hohen Absätzen geeignet sein. Außerdem soll ein einfach und kostengünstig durchzuführendes Zurichtungsverfahren angegeben werden.

Diese Aufgabe wird mit einem orthopädisch zugerichteten Schuh mit den Merkmalen des Anspruchs 1 gelöst.

Vorteilhafte Ausführungsformen sind in den Unteransprüchen angegeben.

Im folgenden wird die Erfindung anhand der Zeichnung beispielhaft näher erläutert. Es zeigen:
Figur 1: eine erste Ausführungsform des erfindungsgemäßen Schuhs in einer Längsschnittdarstellung;
Figur 2: eine Draufsicht auf die Ausführungsform gemäß Figur 1;
Figur 3: Ein Beispiel eines nichterfindungsgemäßen orthopädisch zugerichteten Schuhs in einer Längsschnittdarstellung;
Figur 4: das Beispiel gemäß Figur 3 in einer Draufsicht und
Figur 5: Ein weiteres Beispiel eines nichterfindungsgemäßen orthopädisch zugerichteten Schuhs im Längsschnitt.

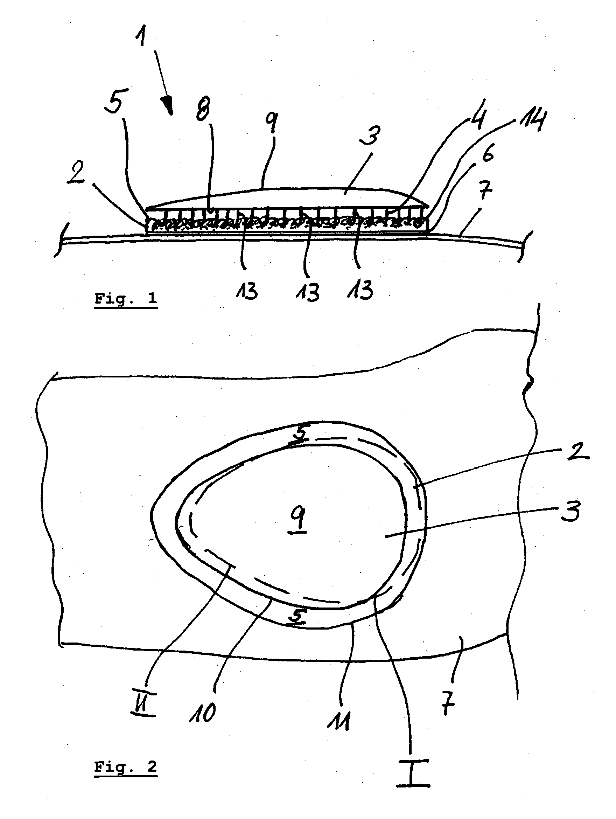

Erfindungsgemäß wurde erkannt, dass eine geeignete Vorrichtung 1 zum orthopädischen Zurichten von Schuhwerk (Fig. 1) lediglich zumindest eine schmiegsame, biegsame, bevorzugt textile Unterlageschicht 2 zum Anordnen auf der Innensohle eines zuzurichtenden Schuhs und zumindest ein Korrekturelement 3, welches mittels einer Klettschicht 4 eine Klettverbindung mit der Unterlageschicht 2 ausbildend auf einer freien Oberseite 5 der Unterlageschicht 2 lösbar befestigbar ist. Die Unterlageschicht 2 ist bevorzugt mittels einer Klebstoffschicht 6 auf einer Innensohle 7 des zuzurichtenden Schuhs befestigt. Die Klebstoffschicht 6 ist entweder bereits an der Unterlageschicht 2 vorkonfektioniert und kann ggf. zweckmäßigerweise mit einer Abdeckschicht, z. B. einem Abdeckpapier, abgedeckt sein. Selbstverständlich ist es auch möglich, die Klebestoffschicht erst kurz vor der Montage der Unterlageschicht 2 händisch aufzubringen. Hierbei hat sich vorteilhafterweise gezeigt, dass die Unterlageschicht 2 mittels handelsüblichen Klebstoffen mit erheblich höherer Dauerfestigkeit auch unter Wärme- und Feuchtigkeitseinwirkung auf verschiedenen Innensohlenmaterialien befestigen lässt. Die Unterlageschicht 2 ist als Gegenklettschicht für eine nachfolgend beschriebene Klettschicht zur Ausbildung einer Klettverbindung ausgebildet.

Das Korrekturelement bzw. die Korrekturelemente 3 einer erfindungsgemäßen Vorrichtung 1 zum orthopädischen Zurichten von Schuhwerk ist beispielsweise als Quergewölbestütze, das heißt z. B. als eine Pelotte, als Längsgewölbestütze, als retrokapitale Abstützung, als Zehenstrecker oder -leiste, als Supinationsleiste und/oder als Pronationsleiste oder dergleichen bekannte Korrekturelemente zur Beeinflussung einer Sohleninnenkontur ausgebildet.

Das Korrekturelement 3 ist ein räumlich dreidimensionaler Körper mit einer bevorzugt ebenen Unterseite 8 und einer konturierten Oberseite 9, wobei die Unterseite 8 im eingebauten Zustand des Korrekturelements 3 der Unterlageschicht 2 zugewandt ist. Das Korrekturelement 3 bzw. die Korrekturelemente 3 sind bevorzugt aus einem Ethylen-Vinyl-Acetat (im folgenden: EVA-Material) hergestellt. Dieses Material hat den Vorteil, dass die Korrekturelemente 3 besonders einfach aus einem plattenförmigen Ausgangsmaterial hinsichtlich ihrer Raumform patientenindividuell spanend hergestellt, z. B. gefräst oder geschliffen werden können. Zudem ermöglicht das EVA-Material eine sehr feste und insbesondere dauerfeste Verklebung mit der Klettschicht 4 wie weiter unten beschrieben.

Das EVA-Material besitzt gute Rückstelleigenschaften, d. h. es ist elastisch und doch formstabil. Weiterhin ist es thermisch verformbar, hautverträglich und gut zu verkleben. Das EVA-Material ist ein geschlossenporiger Schaumstoff, der sich sehr gut zerspanen, insbesondere CNC-Fräsen lässt. Das EVA-Material kann auch in einer Form gebacken werden, was sich insbesondere für hohe Stückzahlen anbietet. Weiterhin ist das EVA-Material wegen seiner Geschlossenporigkeit abweisend für Schmutz, Bakterien und Feuchtigkeit, so dass es auch aus hygienischen Gründen besonders geeignet ist. Zudem ist es waschbar.

Weiterhin besitzt das Korrekturelement 3 eine Umfangskontur 10, deren flächenmäßige Ausdehnung bevorzugt etwas kleiner ist als eine Umfangskontur 11 der Unterlageschicht 2, so dass in einer Draufsicht die Unterlageschicht 2 flächig etwas größer ist als das Korrekturelement 3 und/oder dieses zumindest teilweise überragt. Auf der Unterseite 8 des Korrekturelements 3 ist die Klettschicht 4 angeordnet, welche eine Vielzahl von Verhakungselementen 13 besitzt, die sich mit Gegenverhakungselementen 14, z. B. Schlaufen oder Schlingen der Unterlageschicht 2 auf der freien Oberseite 5 der Unterlageschicht 2 verhaken können. Die Verhakungselemente 13 z. B. können Haken- oder Pilzklettelemente sein und sind bevorzugt als hexagonale Pilze mit einer Dichte von 288 Pilzen pro Quadratzentimeter ausgebildet. Die Klettschicht 4 ist bevorzugt als extrudierter Polyprophlyenklett mit einer Dicke von circa 0,3 mm bis 0,8 mm, insbesondere 0,5 mm bis 0,4 mm ausgebildet.

Eine geeignete Klettschicht 4 weist z. B. ein Flächengewicht von 195 g pro Quadratmeter auf und besitzt einen Einsatztemperaturbereich von -30°C bis +90°C. Eine solche Klettschicht ist beispielsweise bei der Gottlieb Binder GmbH & Co. KG, D-71084 Holzgerlingen unter dem Verkaufsnamen Mikroplast®, Typbezeichnung 45-288-HX200-PP3, Artikelnummer 25445 erhältlich. Dieser sogenannte "Mikroplast®-Klett" hat sich besonders bewährt. Die Klettschicht 4 ist mittels einem auf die Materialkombination des Korrekturelements 3 (bevorzugt EVA) und der Klettschicht 4 (bevorzugt Polypropylen) abgestimmten Klebstoff fest mit dem Korrekturelement 3 verbunden.

Die Unterlageschicht 2 ist aus einem im Vergleich zur Dicke des Korrekturelements 3 dünnen textilen Material, mit einer Dicke von 0,3 mm bis 1,5 mm, insbesondere 0,5 mm bis 1,0 mm ausgebildet und besitzt eine nichtfransende Umfangskontur 11. Wesentlich ist, dass das schmiegsame, biegsame Material der Unterlageschicht 2 zumindest an seiner freien Oberseite 5, die im Einbauzustand dem Korrekturelement 3 zugewandt ist, geeignete Gegenverhakungselemente 14 für die Verhakungselemente 13 der Klettschicht 4 besitzt. Die Gegenverhakungselemente 14 sind hierbei beispielsweise als Schlingen oder Schlaufen ausgebildet.

Als Unterlageschicht 2 kann insbesondere bei der Ausführungsform gemäß Figur 1 und 2 ein sehr dünnes und leichtes textiles Polyamidmaterial mit beispielsweise einem Flächengewicht von 50 g pro Quadratmeter verwendet werden.

Überraschenderweise hat sich gezeigt, dass insbesondere bei Verwendung eines sehr feinen Pilzklettbandes für die Klettschicht 4, z. B. der oben erwähnte Mikroplast®-45288-HX200-PP3-Klett die Klettverbindung gute Verbindungseigenschaften, insbesondere gute Festigkeit auch dann aufweist, wenn die Unterlageschicht 2 ein Gewebe, Gewirke oder ein Filz mit aufgerauter Oberfläche ist. Es hat sich gezeigt, dass eine solche dünne textile Unterlageschicht 2 bereits in der Lage ist, eine ausreichend schubfeste Klettverbindung mit dem Korrekturelement 3 einzugehen. Dies wird darauf zurückgeführt, dass die Klettverbindung zwischen dem Korrekturelement 3 und der Unterlageschicht 2 im Gebrauch zwar Schubbelastungen ausgesetzt ist, gleichzeitig aber auch immer eine ausreichend hohe Andrückung des Korrekturelements 3 auf die Unterlageschicht 2 durch den Patientenfuß erfolgt, so dass eine gute Verkrallung bzw. Verhakung der Verhakungselemente 13 mit den Gegenverhakungselementen 14 der Unterlageschicht 2 erfolgt.

Durch die in der Draufsicht flächenmäßig etwas größere Ausdehnung der Unterlageschicht 2 im Vergleich zur flächigen Ausdehnung des Korrekturelements 3 ist die Möglichkeit eröffnet, die zum Zurichten des Konfektionsschuhs auf die Innensohle 7 zu klebende Unterlageschicht 2 lediglich grob zu positionieren, so dass sie in etwa an der Stelle auf der Innensohle 7 befestigt ist, an der in einem weiteren Schritt das Korrekturelement 3 schlussendlich im zuzurichtenden Schuh angeordnet sein soll. Durch einfaches Aufdrücken des Korrekturelements 3 auf die Unterlageschicht 2 kann dieses mit hoher Schubfestigkeit und insbesondere ausreichend hoher Schubstarrheit relativ zur Unterlageschicht 2 befestigt werden (Position I). Sollte sich nach einem Anprobieren des so zugerichteten Konfektionsschuhs herausstellen, dass die Position des Korrekturelements 3 korrigiert werden muss, so kann durch einfaches Lösen, das heißt Abschälen des Korrekturelements 3 von der Unterlageschicht 2 und erneutem Andrücken auf dieser, in einfacher Art und Weise eine erneute Verbindung zwischen dem Korrekturelement 3 und der Unterlageschicht 2 und somit der Innensohle 7 an einer geänderten Position ausgebildet werden (Position II). Somit muss ein zunächst falsch positioniertes Korrekturelement 3 nicht als Abfall entsorgt werden, sondern kann in einfacher Art und Weise ohne wesentlichen Verlust der Verbindungsqualität nahezu beliebig oft neu positioniert und wieder befestigt werden.

Die Ausführungsform gemäß Figur 1 eignet sich insbesondere für den Einsatzfall, bei dem das Zurichten eines Schuhs nur eines oder wenige Korrekturelemente 3 benötigt und ist insbesondere geeignet zum Anbringen einer Quergewölbestütze (Pelotte) im Schuh.

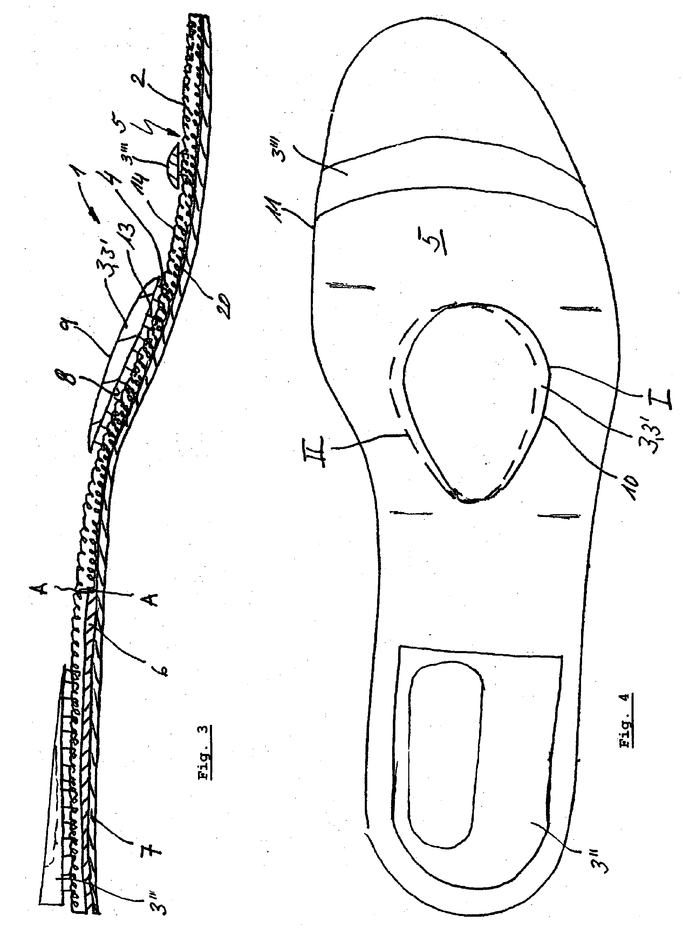

Für das Anbringen mehrerer Korrekturelemente 3 auf der Innensohle 7 eines zuzurichtenden Schuhs, z. B. einer Pelotte im Mittelfußbereich, einem Fersenkissen im Fersenbereich, einem Zehenstrecker bzw. einer Zehenleiste im Zehenbereich oder Supinationsleisten bzw. Pronationsleisten an den Längskantenbereichen der Innensohle, hat sich das im folgenden beschriebene Beispiel gemäß Figur 3 und Figur 4 besonders bewährt.

Im Unterschied zur Ausführungsform gemäß Figur 1 und 2 besitzt die Vorrichtung 1 zum Zurichten von Schuhwerk gemäß Figur 3 und 4 eine biegsame, schmiegsame, z. B. textile Unterlageschicht 2, deren Außenkontur 11 in etwa der Kontur der Innensohle 7 des zuzurichtenden Schuhs entspricht, so dass die Unterlageschicht 2 die Innensohle des zuzurichtenden Schuhs vollständig oder nahezu vollständig bedeckt. Die Unterlageschicht 2 kann hinsichtlich ihres Schichtaufbaus ebenso ausgebildet sein, wie die Unterlageschicht 2 gemäß der Ausführungsform nach Figur 1 und 2, das heißt, sie weist z. B. eine textile Materialschicht auf, welche mittels einer Klebstoffschicht 6 auf der Innensohle 7 des zuzurichtenden Schuhs aufgeklebt ist. Die Klebstoffschicht 6 bedeckt zumindest teilbereichsweise, z. B. randlich umlaufend die Unterlageschicht 2.

Die Unterlageschicht 2 ist bevorzugt als textile Schicht ausgebildet und besteht beispielsweise aus einem kettengewirkten Schlingenvelours und besitzt als Gegenverhakungselemente 14 hochgeraute Schlingen, bevorzugt Schlaufen oder dergleichen. Bevorzugt besteht der Schlingenvelours aus Polyamid und weist eine Dicke von 1,0 mm bis 2,0 mm insbesondere 1,5 mm bis 1,6 mm auf. Ein geeigneter Schlingenvelours hat z. B. ein Flächengewicht von 230 g +/- 20 g pro Quadratmeter und besitzt einen Einsatztemperaturbereich von -30°C bis +100°C. Die im Vergleich zur Ausführungsform gemäß Figur 1 und 2 etwas größere Dicke der textilen Unterlageschicht 2 kann bei der Vorrichtung gemäß Figur 3 bzw. Figur 4 ohne weiteres in Kauf genommen werden, da die textile Unterlageschicht 2 die gesamte Innensohle 7 des zuzurichtenden Schuhs bedeckt und somit keine für den Patientenfuß spürbare Kanten der textilen Unterlageschicht 2 vorhanden sind. Hierdurch ist sichergestellt, dass keine ungewollten stufenförmige Reibungsstellen existieren, die z. B. zur Blasenbildung oder zu scheuernden Verletzungen des Patientenfußes führen können. Gleichwohl ist die textile Unterlageschicht 2 immer noch so dünn, dass kein nennenswerter Verlust an Tragekomfort, auch in modischem, z. B. eng geschnittenem Schuhwerk auftritt. Außerdem ist die textile Unterlageschicht 2 über ihre gesamte Flächenerstreckung biegsam und schmiegsam, so dass sich diese im Gegensatz zu einer zumindest teilweise relativ starr ausgebildeten Fußeinlage über die gesamte Flächenerstreckung an die Innensohle 7 des zuzurichtenden Schuhs ohne Bildung von Hohlräumen zwischen der Unterlageschicht 2 und der Innensohle des Schuhs anschmiegt.

In einem weiteren Beispiel (in Figur 3 rechts von der angedeuteten Linie A-A dargestellt) ist die Unterlageschicht 2 als sogenannter back-to-back-velours ausgebildet, das heißt, dass sowohl die freie Oberseite 5 der textilen Unterlageschicht 2 als auch deren gegenüberliegende Unterseite 20, die im eingebauten Zustand der Innensohle 7 zugewandt ist, ebenfalls Schlingen, Schlaufen oder dergleichen Gegenverhakungselemente 14 aufweist. Bei dieser Ausgestaltung der textilen Unterlageschicht 2 hat sich überraschenderweise gezeigt, dass insbesondere in relativ flachem Schuhwerk bei einer genauen Einpassung der textilen Unterlageschicht 2 in den Innenschuh des zuzurichtenden Schuhs die Klebeschicht 6 sogar entfallen kann. Ein geeignetes textiles Material für die Unterlageschicht 2 ist beispielsweise bei der Gottlieb Binder GmbH & Co. KG, D-71084 Holzgerlingen unter der Artikelnummer 88297 erhältlich.

Auf der textilen Unterlageschicht 2 können eine Vielzahl von Korrekturelementen 3, z. B. eine Pelotte 3', ein Fersenkissen 3'', eine Zehenleiste 3''' oder dergleichen andere geeignete Korrekturelemente, wie sie oben bereits beschriebenen wurden, mittels der Klettschicht 4 in einfacher Art und Weise aufgesetzt und befestigt werden. Sollte eine Korrektur der Position oder ein Ersatz eines der Korrekturelemente 3 erforderlich sein, so können die Korrekturelemente 3 in einfacher Art und Weise durch Lösen der Klettverbindung (Position I) und erneutes Andrücken in ihrer Position (Position II) geändert werden oder durch andere Korrekturelemente 3 ersetzt werden, ohne dass das zu versetzende oder zu ersetzende Korrekturelement 3 zerstört wird.

Als Klebstoff für die Unterlageschicht 2 hat sich im Rahmen der Erfindung sowohl für die Ausgestaltung gemäß der Ausführungsform 1 und 2 als auch für die Ausgestaltung gemäß der Ausführungsform gemäß der Figuren 3 und 4 ein UV-vernetzter Acrylat-Klebstoff bewährt.

Gemäß einer weiteren nicht-erfindungsgemäßen Vorrichtung ist die textile Unterlageschicht 2 mit ihrer Unterseite 20 auf einer Trägerschicht 21 angeordnet und mit dieser verbunden. Die Trägerschicht 21 ist aus einem dünnen plattenartigen Ethylen-Vinyl-Acetat Material (EVA-Material) hergestellt. Die oben bereits aufgeführten Vorteile betreffend das EVA-Material gelten in gleicher Weise auch für die Trägerschicht 21. Insbesondere ist es in einfacher Art und Weise möglich, die Trägerschicht randlich umlaufend mit einer Anfasung 22, beispielsweise spanend oder schleifend zu versehen, so dass ein angenehmer optischer Eindruck entsteht und zudem durch die Anfasung ein erleichtertes Einlegen der erfindungsgemäßen Vorrichtung in einen zuzurichtenden Schuh ermöglicht ist. Die textile Unterlageschicht ist bevorzugt mit der Trägerschicht 21 flächig verbunden, insbesondere ist die textile Unterlageschicht 2 auf der Trägerschicht 21 aufkaschiert. Ebenso eignen sich flächig wirkende Verklebungsmethoden, wie z. B. den Klebstoffauftrag in einem Punkt- und/oder Streifen- und/oder Gitterraster sowie ein vollständiger vollflächiger Klebstoffauftrag zwischen der textilen Unterlageschicht 2 und der Trägerschicht 21.

Die Trägerschicht 21 besitzt bevorzugt eine Dicke zwischen 0,5 und 4 mm, insbesondere zwischen 1,0 mm und 3 mm. Die textile Unterlageschicht 2 entspricht hinsichtlich ihrer Materialwahl der der vorbeschriebenen Ausführungsbeispiele.

Weiterhin kann es zweckmäßig sein, die Korrekturelemente 3 an deren Unterseite nur teilweise mit der Klettschicht 14 zu versehen. Beispielsweise hat es sich als sinnvoll erwiesen, eine Pelotte zum Einsatz in der erfindungsgemäßen Vorrichtung unterseitig nur zu etwa 1/3 bis 2/3 mit der Klettschicht 14 zu versehen, wobei die Klettschicht 14 in dem Teilbereich der Unterseite der Pelotte angebracht ist, die im bestimmungsgemäßen Gebrauch zur Ferse des Patienten weist. Eine geeignete Bedeckung der Unterseite der Pelotte mit der Klettschicht ist beispielsweise ein 1/4 bis 3/4 , insbesondere 1/3 bis 2/3 der Unterseite. Hieraus resultiert der Vorteil, dass bei einer Pelotte der vordere Bereich unverklettet mit der textilen Unterlageschicht 2 verbleibt und somit im Falle des Notwendigwerdens des Versetzens der Pelotte auf der textilen Unterlageschicht 2 diese leicht gegriffen werden kann, so dass die Pelotte leicht von der Unterlageschicht abgenommen werden kann. Selbstverständlich liegt es auch im Bereich der Erfindung, sämtliche Korrekturelemente 3, 3', 3'', 3''' als ein kombiniertes Korrekturelement auszubilden, in welchem eine oder mehrere der folgenden Funktionsbestandteile integriert ausgebildet sind. Mögliche Funktionsbestandteile sind die oben bereits genannten Längsgewölbestützen, Fersenkeile, Quergewölbestützen, wobei ggf. an geeigneten Stellen Fersenspornaussparungen integriert sind.

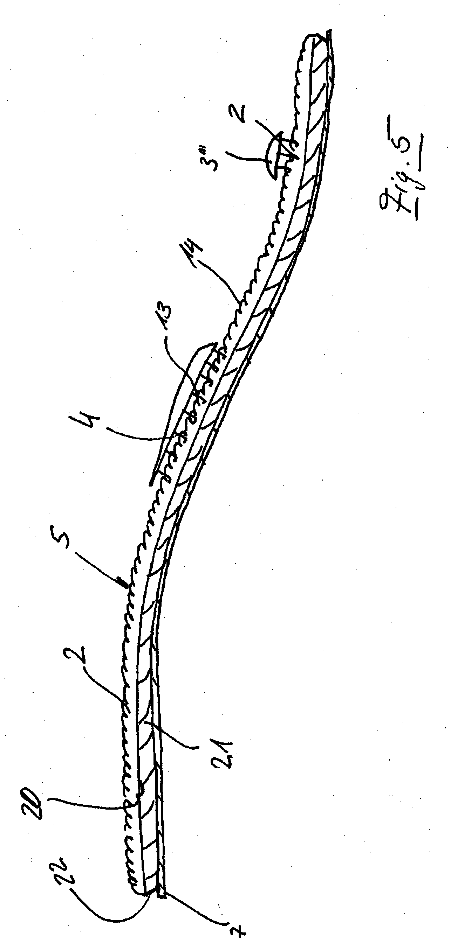

Ein weiterer Vorteil der Vorrichtung gemäß Figur 5 ist, dass der Schichtaufbau bestehend aus der textilen Unterlageschicht 2 zusammen mit der Trägerschicht 7 ohne weiteres in üblichen Waschmaschinen waschbar ist, ohne das eine unerwünschte Änderung der Raumform der Vorrichtung auftritt.

Somit bietet ein erfindungsgemäßer konfektionierter Schuh gegenüber dem handwerklichen Schuh, wie es in der Beschreibungseinleitung beschrieben wurde einen erheblichen Kostenvorteil in Höhe von bis zu 70% bis 90%. Zudem wird eine einfache und unkomplizierte Korrekturmöglichkeit der Positionen (Position I → Position II) der Korrekturelemente 3 im zuzurichtendem Schuh gewährleistet, die vom Patienten selbst ohne handwerklichen Aufwand eines Orthopädiemechanikers bewerkstelligt werden kann.

Gleichzeitig sind mit dem erfindungsgemäßen Schuh die Nachteile des aufwändigen handwerklichen Bearbeitens überflüssig und die Befestigung der Korrekturelemente 3 mit ausreichend hoher Festigkeit möglich.

Außerdem ist der erfindungsgemäß zugerichtete Schuh durch seine schmiegsame, biegsame Unterlage auch zum Zurichten bzw. auch von Schuhwerk mit hohen Absätzen, d. h. mit hoher Sprengung geeignet.

Durch die leichte Biegbarkeit der Vorrichtung wird das Abrollverhalten des Schuhs nicht beeinflusst, so dass der Tragekomfort erhöht ist.

Die gewollte, z. B. punktuelle oder linienförmige Entlastung bzw. Unterstützung des Patientenfußes erfolgt somit definierter und genauer als mit einer Fußeinlage.

Selbstverständlich liegt es auch im Bereich der Erfindung, die Gegenklettschicht an der Unterseite des/der Korrekturelemente 3 anzuordnen und die Unterlageschicht 2 als Klettschicht mit Verhakungselementen auszubilden.

## Patentansprüche

1. Orthopädisch zugerichteter Schuh, auf dessen Innensohle (7) eine als Gegenklettschicht zur Bildung einer Klettverbindung mit einer Klettschicht (4) ausgebildete Unterlageschicht (2) angeordnet ist und der zumindest ein Korrekturelement (3) aufweist, welches auf dessen Unterseite (8) eine Klettschicht (4) aufweist und mittels der Klettschicht (4) auf einer freien Oberseite (5) der Unterlageschicht (2) zerstörungsfrei lösbar befestigt ist,
**dadurch gekennzeichnet,**
**dass** die Unterlageschicht (2) die Innensohle (4) des Schuhs nicht vollständig und nicht nahezu vollständig bedeckt, wobei eine Umfangskontur (11) der Unterlageschicht (2) eine Umfangskontur (10) des Korrekturelements (3) in einer Draufsicht überragt, das heisst flächig etwas grösser ist, dass die Unterlageschicht (2) aus einem dünnen textilen Material mit einer Stärke von zwischen 0,3 bis 1,5 mm ausgebildet ist, und dass die Unterlageschicht (2) mit einer Klebstoffschicht (6) auf der Innensohle (7) befestigt ist.

2. Schuh nach Anspruch 1, **dadurch gekennzeichnet, dass** die Unterlageschicht (2) biegsam und/oder schmiegsam ist.

3. Schuh nach Anspruch 1 oder 2, **dadurch gekennzeichnet, dass** die Unterlageschicht (2) aus einem Gewebe, Gewirke oder einem Filz ausgebildet ist.

4. Schuh nach einem der vorangegangenen Ansprüche, **dadurch gekennzeichnet, dass** die Klettschicht (4) ein Hakenklett oder ein Pilzklett ist.

5. Schuh nach einem der vorangegangenen Ansprüche, **dadurch gekennzeichnet, dass** das Korrekturelement (3) eine Quergewölbestütze, eine Längsgewölbestütze, eine Supinationsleiste, eine Pronationsleiste oder ein derartiges Bettungs- und/oder Stützungselement ist.

6. Schuh nach einem der vorangegangenen Ansprüche, **dadurch gekennzeichnet, dass** das Korrekturelement (3) aus einem Ethylen-VinylAcrylat-Material hergestellt ist.

## Claims

1. Orthopedically finished shoe on whose insole (7) a foundation layer (2) is arranged, which is formed as counter velcro layer to form a velcro connection with a velcro layer (4), and which includes at least one correction element (3) which on its underside (8) includes a velcro layer (4) and by means of the velcro layer (4) is fixed on a free upper side (5) of the foundation layer (2) to be releasable in a non-destructive way,
**characterized in**
**that** the foundation layer (2) does not completely and not almost completely cover the insole (4) of the shoe, wherein a circumferential contour (11) of the foundation layer (2) in a top view protrudes beyond a circumferential contour (10) of the correction element (3), i.e. has a slightly larger surface area, that the foundation layer (2) is formed of a thin textile material with a thickness of between 0.3 to 1.5 mm, and that the foundation layer (2) is fixed on the insole (7) with an adhesive layer (6).

2. The shoe according to claim 1, **characterized in that** the foundation layer (2) is flexible and/or supple.

3. The shoe according to claim 1 or 2, **characterized in that** the foundation layer (2) is formed of a woven fabric, knit fabric or a felt.

4. The shoe according to any of the preceding claims, **characterized in that** the velcro layer (4) is a hook velcro or a mushroom velcro.

5. The shoe according to any of the preceding claims, **characterized in that** the correction element (3) is a transverse arch support, a longitudinal arch support, a supination strip, a pronation strip or such bedding and/or support element.

6. The shoe according to any of the preceding claims, **characterized in that** the correction element (3) is made of an ethylene-vinyl-acrylate material.

## Revendications

1. Chaussure à préparation orthopédique, sur la semelle intérieure (7) de laquelle est agencée une sous-couche (2) réalisée sous forme de contre-couche aggripante pour former une attache auto-aggripante avec une couche aggripante (4), et laquelle présente au moins un élément de correction (3) qui présente sur sa face inférieure (8) une couche aggripante (4) et qui est fixé de manière détachable et non destructive sur une face supérieure (5) libre de la sous-couche (2) au moyen de la couche aggripante (4),
**caractérisée en ce que**
la sous-couche (2) ne recouvre pas totalement ni pas presque totalement la semelle intérieure (4) de la chaussure, un contour périphérique (11) de la sous-couche (2) dépassant un contour périphérique (10) de l'élément de correction (3) en vue de dessus, c'est-à-dire que sa surface est un peu plus grande, **en ce que** la sous-couche (2) est constituée par un matériau textile mince avec une épaisseur entre 0,3 et 1,5 mm, et **en ce que** la sous-couche (2) est fixée sur la semelle intérieure (7) par une couche de colle (6).

2. Chaussure selon la revendication 1, **caractérisée en ce que** la sous-couche (2) est flexible et/ou souple.

3. Chaussure selon la revendication 1 ou 2, **caractérisée en ce que** la sous-couche (2) est réalisée en tissu, en tricot ou en feutre.

4. Chaussure selon l'une des revendications précédentes, **caractérisée en ce que** la couche aggripante (4) est une couche à crochets ou une couche à champignons.

5. Chaussure selon l'une des revendications précédentes, **caractérisée en ce que** l'élément de correction (3) est un soutien de voute transversale, un soutien de voute longitudinale, un insert de supination, un insert de pronation ou un tel élément de semelle et/ou de soutien.

6. Chaussure selon l'une des revendications précédentes, **caractérisée en ce que** l'élément de correction (3) est réalisé en un matériau d'éthylène-acrylate de vinyle.
